# EUROPEAN PATENT APPLICATION

(11) **EP 4 799 673 A1**
(43) Date of publication of application: **02.09.2026**
(21) Application number: 24881829.6
(22) Date of filing: 28.10.2024
(51) Int. Cl.: A61M 39/22, A61M 60/37, A61M 60/851

(54) **DEVICE FOR CHANNELLING BLOOD FOR HAEMODIALYSIS**

(30) Priority: 27.10.2023 ES 202330882
(71) Applicant: Fundación para la Investigación del Hospital Clínico de la Comunidad Valenciana (INCLIVA), 46010 Valencia (ES); Klus Life Systems, S.L.U., 28046 Madrid (ES); Universitat de València, 46010 Valencia (ES)
(72) Inventor: CABALLERO VÁRON, Valeriano, 28046 Madrid (ES); MEDINA BASSO, Pascual, 46010 Valencia (ES); GARCIA GIMENEZ, José Luis, 46010 Valencia (ES); BAHAMONDE ROMANO, José Angel, 46010 Valencia (ES)
(74) Representative: Ibarra Garcia, Isabel
(86) International application number: PCT/ES2024/000025
(87) International publication number: WO 2025/088229

(57) **Abstract**

The present invention relates to a device for channelling blood for haemodialysis comprising a bypass valve (2), with two vascular connection ducts (3, 4), and a connection (5) to a dialyser. The valve (2) comprises an inner selector (6) for selecting two positions and an antiseptic reservoir (7) placed in the connection (5) to a dialyser, as well as biointegration means.

## Description

### OBJECT OF THE INVENTION

The present invention relates to a device for channelling blood for haemodialysis, including for blood transfusions or extractions.

### BACKGROUND OF THE INVENTION

Chronic kidney disease (CKD) is one of the so-called silent diseases. When a patient suffers from it, they have only two alternatives: to receive a transplant or to undergo dialysis. As there are not enough organs for everyone (in addition to other determining factors such as compatibility), it is necessary to find adequate vascular access for dialysis, which is generally performed by means of a catheter, an arteriovenous graft (AVG) or an arteriovenous fistula (AVF), which is the option preferred by specialists. AVF consists of the surgical attachment of a vein and an artery, and after the operation, its functioning is not guaranteed until after a maturation period (2 to 4 weeks) and its functioning has been checked. This technique has not changed since 1960 and can cause side effects such as stenosis, aneurysms, clots and even psychological problems, which are avoided with the use of the device of the invention.

### DESCRIPTION OF THE INVENTION

In a generic description, the device for channelling blood for haemodialysis of the invention comprises a bypass valve, with two vascular connection ducts for connecting same to the vessel where it is to be implanted (vein or artery), and a connection to a dialyser to bypass blood through same. Said valve comprises an inner selector for selecting two positions (closed, where there is continuity between ducts with the connection to a dialyser closed, or open, where there is continuity between a non-closable duct and the connection to a dialyser, while the other closable duct remains closed), as well as an antiseptic reservoir placed in the connection to a dialyser, also comprising biointegration means. It also preferably has holes for suturing the device to the patient, designed for a 1/2 circle surgical needle. Similarly, it has blades for the biointegration of the device and for the purpose of preventing internal displacements and rotations when being handled by the personnel in charge of making the connection following clinical recommendations.

Therefore, and as a result of breakthroughs made in the field of the biomaterial engineering and new manufacturing techniques, it is possible to make the device of the invention and solve the problems in the current art, since the device allows an orderly, homogeneous and continuous flow to be obtained both at the inlet and at the outlet of the dialyser. In this sense, AVF maturation times and venous and arterial deformations occurring in AVFs for different reasons are prevented. Moreover, it must be noted that it is not necessary to constantly puncture the patient as occurs today, since the device can be implanted in different vascular areas, being completely aseptic when it is closed as a result of the arrangement of an antiseptic in the reservoir for that purpose, thereby ensuring the prophylaxis thereof. It can also be used with other diseases causing vascular pathologies, such as cancer or diabetes mellitus, and in addition to the aforementioned, it can also be used for blood transfusions or extractions.

In general, it solves all the problems posed by current techniques, such as wait times (none), vascular deformations (non-deformable), implant location problems (any location is acceptable), or infections, among others.

### BRIEF DESCRIPTION OF THE DRAWINGS

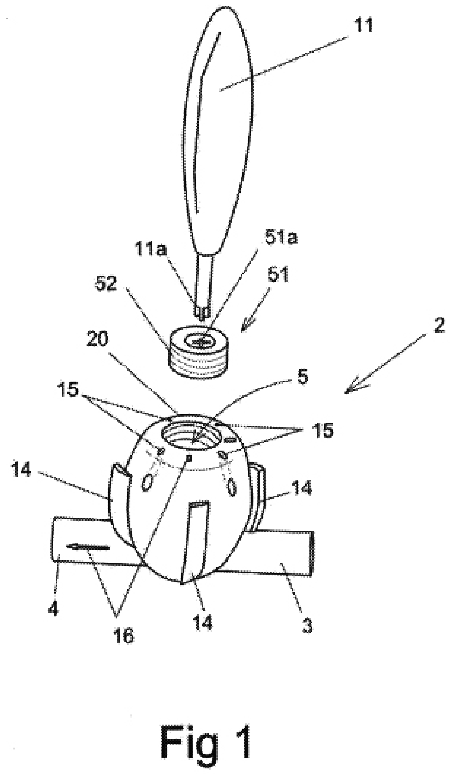

Figure 1 shows an outer view of the valve of the device of the invention, with the closure cap and opener thereof.

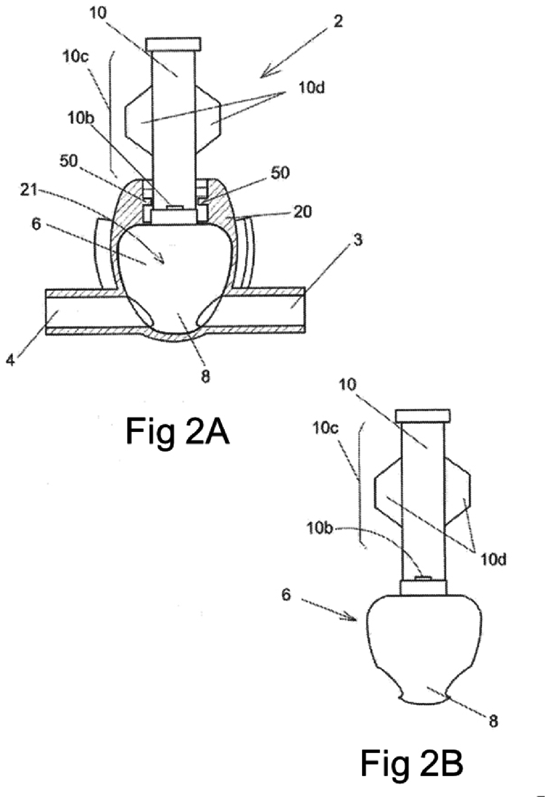

Figure 2A shows a section of the body of the valve, where the relative position of the selector (without sectioning) can be seen in the position of continuity between both ducts and with the connector in place before rotating same a quarter turn; whereas Figure 2B shows a detail of the selector in the same position, also with the connector in place. In this position, the device is closed so it is not possible to perform dialysis and the flow proceeds naturally.

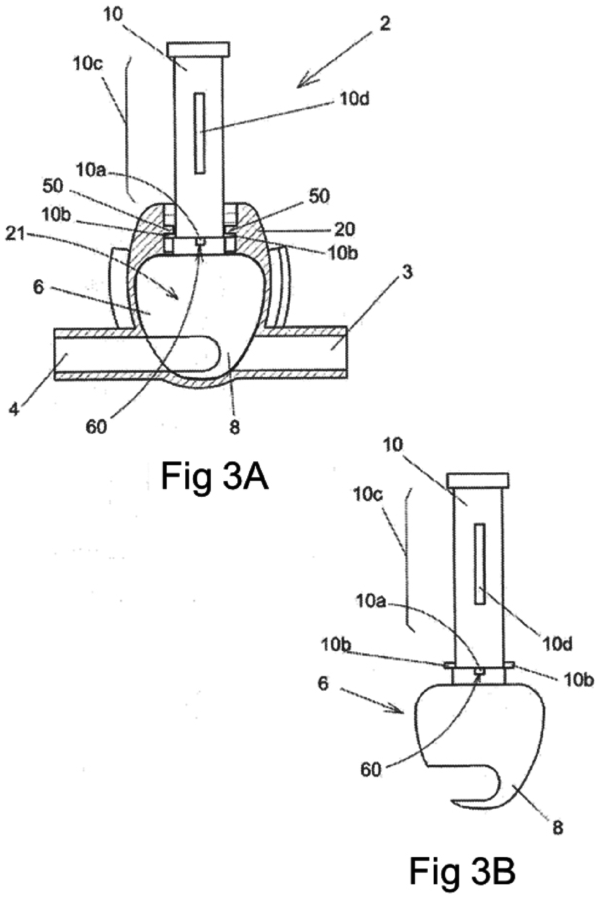

Figure 3A shows a section of the body of the valve where the relative position of the selector (without sectioning) can be seen in the bypass position between the non-closable duct and the connection to a dialyser, and with the connector in place after rotating same a quarter turn; whereas Figure 3B shows a detail of the selector in the same position and with the connector in place. In this position, the device is open so it is possible to perform dialysis or return the blood from the dialyser to the vein. In this position, it is possible to perform blood extractions or transfusions.

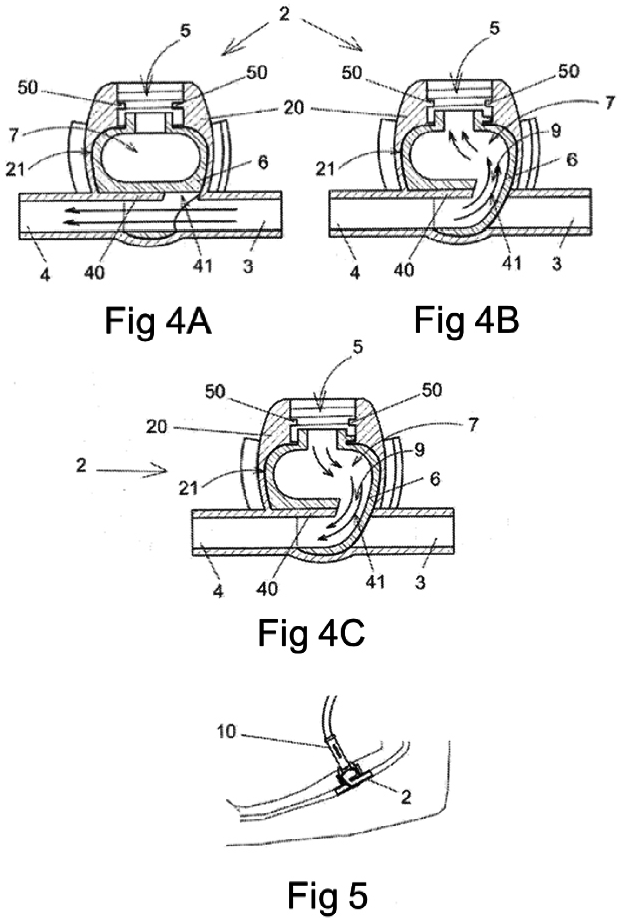

Figure 4A shows a sectioned view of the valve (including the body and the selector) in the position of continuity between both ducts, without the connector, and with the reservoir ready to be filled with antiseptic. In this position, the device is closed so it is not possible to perform dialysis and the flow proceeds naturally in the vessel where it has been implanted.
Figure 4B shows a sectioned view of the valve (including the body and the selector) in the bypass position between the non-closable duct and the connection to a dialyser during extraction without the connector. In this position, the device is open so it is possible to perform dialysis or return the blood from the dialyser to the vein. In this position, it is possible to perform blood extractions.
Figure 4C shows a sectioned view of the valve (including the body and the selector) in the bypass position between the non-closable duct and the connection to a dialyser during infusion without the connector. In this position, the device is open so it is possible to perform dialysis or return the blood from the dialyser to the vein. In this position, it is possible to perform blood transfusions.
Figure 5 shows a view of the implantation of the device in a forearm, connecting the ducts to a vein.

### DESCRIPTION OF A PRACTICAL EMBODIMENT OF THE INVENTION

The device for channelling blood for haemodialysis of the invention (see Figures 1 and 5) comprises a bypass valve (2), with two vascular connection ducts (3, 4), and a connection (5) to a dialyser. The valve (2) comprises an inner selector (6) (see Figures 2A to 4C) for selecting two positions and communicated, by means of the connection (5) and a connector (10) connected therein, to the dialyser, or to the extraction or transfusion. It also comprises an antiseptic reservoir (7) placed in the connection (5) to a dialyser, as well as biointegration means, ideally consisting of four blades (14).

The reservoir (7) is integrated in the selector (6), as seen in Figures 4A to 4C, since that simplifies the configuration of the device and, therefore, the manufacture thereof. In this case, the bypass valve (2) preferably comprises a body (20) with an inner cavity (21) into which the two ducts (3, 4) open in the lower portion and the connection (5) to a dialyser opens in the upper portion; there being rotatably arranged in said cavity (21) the selector (6), which comprises the reservoir (7) on the area of communication of the ducts (3, 4) with the inner cavity (21) of the body (20) of the valve (2), as well as a lower lateral plugging segment (8) in the area of communication of the ducts (3, 4), with a breadth equal to or greater than the lumen of the closable duct (3), and furthermore a first opening (9) in the lower lateral part of the reservoir (7) (see Figures 4B and 4C); with the non-closable duct (4) comprising an extension (40) extending into the cavity of the body which determines a second opening (41) offset from the first opening (9) in the position of continuity of flow between the two ducts (3, 4) as seen in Figure 4A (and therefore blocking the flow towards the connection (5)), and aligned with the first opening (9) in the position of communication of the non-closable duct (4) with the connection (5) to a dialyser as seen in Figures 4B and 4C. Taking the above into account, when the selector is in the closed position (Figures 2A and 4A), it allows the circulation of the flow in its natural direction, whereas when it is open (Figures 3A, 4B and 4C), it allows the extraction or infusion of the flow. In addition to the above and in the closed position, it allows providing antiseptic in the reservoir (7) to ensure prophylaxis.

The arrangement of the detachable tubular connector (10), insertable into the connection (5) to a dialyser, has also been envisaged in order to connect the patient to the dialyser without punctures (see Figures 2A to 3B and Figure 5).

Likewise, the selector (6) preferably comprises a first actuating mechanism (60) (see Figures 3A and 3B) (for rotating and placing the valve in one operating mode or the other); the connector (10) comprising a tip (10a) with a shape complementary to said first mechanism (60). This allows, when performing dialysis, the connector to be inserted and the valve to thereby be placed directly in the transfusion position. Furthermore, the connector (10) preferably comprises radial flanges (10b), and the connection (5) to a dialyser preferably comprises stops (50) for said flanges (10b) in the bypass position of the selector to prevent the accidental extraction of the connector and bleeding during the transfusion.

Additionally, the connector (10) comprises an outer extension (10c) and handling projections (10d) to facilitate handling.

Furthermore, the connection (5) to a dialyser comprises (see Figure 1) a closure cap (51) to close the access and to prevent the antiseptic from leaking out when it is in the reservoir with the valve in the closed position. Said cap (51) comprises a closure thread (52) and a second actuating mechanism (51a); comprising an opening tool (11) provided with an end (11a) with a shape complementary to said second mechanism (51a) so as to be able to open and close same.

In turn, the biointegration means are selected from:
- protrusions or blades (14) for adipocyte adhesion, which ensures biointegration of the device and prevents internal rotation in the implant area when being handled by the specialist;
- suture holes (15), which preferably have a curved path adapted to a ½ circle surgical needle.

Furthermore, it externally comprises indicators (16) indicating the position of the valve and the internal flow direction.

There is no single material from which the device can be manufactured; therefore, considering that the device is an implantable medical application, it must be ensured that it is safe and effective, and there are countless biomaterials on the market with which it can be manufactured (metals, alloys, polymers, ceramics, etc.) that have currently been tested *in vivo.*

However, the material used to manufacture the device must meet the following requirements:
- Biocompatibility: The material must not trigger adverse immune responses or cause toxicity in the human body. It must be tolerated by the immune system and not cause rejection.
- Chemical and biological stability: It must maintain its integrity and properties in the biological environment of the human body without degrading or releasing toxic substances.
- Mechanical strength: It must be strong enough to withstand the loads and stresses to which it will be exposed in its specific location within the body.
- Suitable surface: It must have a surface that allows suitable interaction with surrounding cells and tissues. This may include adhesion properties and controlled porosity.
- Compatibility with manufacturing techniques: The material must be suitable for manufacturing processes that allow the creation of accurate and safe medical devices.
- Quality control and sterilisation: It must be possible to produce the material in accordance with high quality standards and sterilise it suitably to prevent infection.
- Long-term durability: It must maintain its properties and functionality over time, as some implants may remain in the body for many years.

In any case, it should preferably be manufactured from polymeric or ceramic materials, as both the material and manufacturing costs are lower.

Having sufficiently described the nature of the invention as well as the manner of carrying it out to practice, it should be noted that the arrangements indicated above and depicted in the attached drawings are susceptible to modifications of detail provided that they do not alter the main principle.

## Claims

1. A device for channelling blood for haemodialysis, **characterised in that** it comprises a bypass valve (2), with two vascular connection ducts (3, 4), and a connection (5) to a dialyser; the valve (2) comprising an inner selector (6) for selecting two positions and an antiseptic reservoir (7) placed in the connection (5) to a dialyser, as well as biointegration means.

2. The device for channelling blood for haemodialysis according to claim 1, **wherein** the reservoir (7) is integrated in the selector (6).

3. The device for channelling blood for haemodialysis according to claim 2, **wherein** the bypass valve (2) comprises a body (20) with an inner cavity (21) into which the two ducts (3, 4) open in the lower portion and the connection (5) to a dialyser opens in the upper portion; there being rotatably arranged in said cavity (21) the selector (6), which comprises the reservoir (7) on the area of communication of the ducts (3, 4) with the inner cavity (21) of the body (20) of the valve (2), a lower lateral plugging segment (8) in the area of communication of the ducts (3, 4), with a breadth equal to or greater than the lumen of the closable duct (3), and a first opening (9) in the lower lateral part of the reservoir (7); with the non-closable duct (4) comprising an extension (40) extending into the cavity of the body which determines a second opening (41) offset from the first opening (9) in the position of continuity of flow between the two ducts (3, 4), and aligned with the first opening (9) in the position of communication of the non-closable duct (4) with the connection (5) to a dialyser.

4. The device for channelling blood for haemodialysis according to any of the preceding claims, **comprising** a detachable tubular connector (10), insertable into the connection (5) to a dialyser.

5. The device for channelling blood for haemodialysis according to claim 4, **wherein** the selector (6) comprises a first actuating mechanism (60); the connector (10) comprising a tip (10a) with a shape complementary to said first mechanism (60).

6. The device for channelling blood for haemodialysis according to claim 4 or 5, **wherein** the connector (10) comprises an outer extension (10c) and handling projections (10d).

7. The device for channelling blood for haemodialysis according to any of the preceding claims, **wherein** the connector (10) comprises radial flanges (10b), and the connection (5) to a dialyser comprises stops (50) for said flanges (10b) in the bypass position of the selector.

8. The device for channelling blood for haemodialysis according to any of the preceding claims, **wherein** the connection (5) to a dialyser comprises a closure cap (51).

9. The device for channelling blood for haemodialysis according to claim 8, **wherein** the cap (51) comprises a thread (52) and a second actuating mechanism (51a); comprising an opening tool (11) provided with an end (11a) with a shape complementary to said second mechanism (51a).

10. The device for channelling blood for haemodialysis according to any of the preceding claims, **wherein** the biointegration means are selected from:
- protrusions or blades (14) for adipocyte adhesion,
- suture holes (15).

11. The device for channelling blood for haemodialysis according to claim 10, **wherein** the suture holes (15) have a curved path adapted to a ½ circle surgical needle.

12. The device for channelling blood for haemodialysis according to any of the preceding claims, externally **comprising** indicators (16) indicating the position of the valve and the internal flow direction.
